# EUROPÄISCHE PATENTSCHRIFT

(11) **EP 4 510 954 B1**
(45) Veröffentlichungstag und Bekanntmachung des Hinweises auf die Patenterteilung: **29.07.2026**
(21) Anmeldenummer: 23716906.5
(22) Anmeldetag: 12.04.2023
(51) Int. Cl.: A61B 17/50, A61M 1/00, A61M 16/06, A61B 17/24, A61M 39/24

(54) **VORRICHTUNG ZUM ENTFERNEN VON DEN ATEMLUFTWEG VERSCHLIESSENDEN GEGENSTÄNDEN**
DEVICE FOR REMOVING OBJECTS WHICH OBSTRUCT AN AIRWAY
DISPOSITIF POUR ÉLIMINER DES OBJETS OBSTRUANT LES VOIES RESPIRATOIRES

(30) Priorität: 19.04.2022 DE 202022102066 U
(43) Veröffentlichungstag der Anmeldung: 26.02.2025
(73) Patentinhaber: Jespira GmbH, 44227 Dortmund (DE)
(72) Erfinder: CHEHADE, Nabil, 75417 Mühlacker (DE)
(74) Vertreter: Fleck, Hermann-Josef
(86) Internationale Anmeldenummer: PCT/EP2023/059529
(87) Internationale Veröffentlichungsnummer: WO 2023/202924

(56) Entgegenhaltungen:
- WO-A1-2012/107882
- WO-A1-2021/252376
- CN-A- 107 432 969
- DE-A1- 4 320 815
- US-B1- 10 675 393

## Beschreibung

Die Erfindung bezieht sich auf eine Vorrichtung zum Entfernen von den Atemluftweg verschließenden Gegenständen mittels Saugwirkung, mit einer auf das Gesicht aufsetzbaren, den Mund und/oder die Nase zumindest weitgehend dicht umgebenden Saugmaske und einer an diese zum Bilden einer Saugeinheit dicht angeschlossenen oder anschließbaren, manuell betätigbaren Vakuumpumpe, die einen in einem Hohlraum entlang einer diesen umgebenden Wandfläche geführten, von Hand mittels eines Verbindungsstücks verschiebbaren Kolben aufweist, der beim Einschieben in den Hohlraum Luft aus dem Hohlraum über eine in die äußere Atmosphäre führende Ablasskanalanordnung verdrängt und beim Ausziehen im Hohlraum bei angelegter Saugmaske Unterdruck im Plenum aus einem dieser zugekehrten Hohlraumteil, Saugmaskeninnenraum und anschließendem Atemluftweg erzeugt.

Eine Vorrichtung zum Entfernen von den Atemluftweg verschließenden Gegenständen mittels Saugwirkung dieser Art ist in der CN 107 432 969 A angegeben. Eine Ventilanordnung ist dabei in einem einer Saugmaske zugewandten Wandfläche des Hohlraums angeordnet.

Eine handbetätigte Vorrichtung zum Entfernen von Gegenständen, nämlich Flüssigkeit, aus dem Atem luftweg, z. B. einer Nasenhöhle, ist in der WO 2012 / 107882 A1 gezeigt. Eine Ablasskanalanordnung ist über eine in einer Ringnut eines Kolbens eingelegte Dichtung gebildet. Eine Vergrößerung des Hohlraums zum Absaugen wird durch eine Druckfeder bewirkt.

Auch die DE 43 20 815 A1 zeigt eine handbetätigte Vorrichtung zum Entfernen von Gegenständen aus dem Atemluftweg mittels Saugwirkung.

Eine weitere handbetätigte Absaugvorrichtung mit Saugmaske zeigt die WO 2021 / 252376 A1.

Eine Vorrichtung zum Entfernen von den Atemluftweg verschließenden Gegenständen, mittels der ein in die Luftröhre oder anschließende Teile des Atemwegs gelangter Gegenstand mittels Saugwirkung entfernt wird, ist in der WO 2021/252376 A1 als bekannt ausgewiesen. Hierbei ist an eine auf das Gesicht einer Person abgedichtet über die Nase und den Mund aufsetzbaren Saugmaske eine Vakuumpumpe angeschlossen, um in dem im Rachenraum mit dem Atemweg, dem Innenraum der Saugmaske und dem anschließenden Hohlraumteil der Vakuumpumpe gebildeten Plenum einen Unterdruck zu erzeugen, durch den der den Atemluftweg verschließende Gegenstand angesaugt werden soll, um den Atemluftweg zu öffnen. Die Vakuumpumpe weist einen in einem Hohlraum angeordneten Kolben auf, der entlang der den Hohlraum umgebenden Wandfläche hin- und herbewegbar ist, um beim Ausziehen unter genügender Abdichtung zwischen Kolben und Wandfläche in dem Plenum ein Vakuum zu erzeugen und den Gegenstand aus dem Atemluftweg zu saugen, um diesen zu öffnen. Damit sich beim Einschieben des Kolbens in den Hohlraum in dem Plenum kein zu großer Druck aufbaut und den Gegenstand noch weiter in die Luftröhre bzw. in einen anschließenden Atemkanal zwängt, ist in der Hohlraumwandung zwischen dem plenumseitigen Hohlraumteil und der äußeren Atmosphäre ein Ausströmkanal gebildet, in den ein Einwegventil eingesetzt ist, das ein Ausströmen der Luft aus diesem Hohlraumteil beim Einschieben des Kolbens zulässt, jedoch beim Ausziehen des Kolbens schließt, um Unterdruck bzw. Vakuum zu erzeugen. Das Einwegventil ist beispielsweise als Entenschnabelventil ausgebildet.

Eine weitere Vorrichtung zum Entfernen eines Gegenstands aus dem Atemluftweg ist in der WO 2015/167709 A1 gezeigt. Hierbei ist eine Vakuumpumpe mittels eines Faltenbalgs gebildet und ebenfalls an eine auf das Gesicht einer zu behandelnden Person dichtend aufsetzbaren Saugmaske angeschlossen. Zum Vermeiden eines Überdrucks beim Zusammenschieben des Faltenbalgs ist ein Luftauslassventil in einer Wandung des Faltenbalgs oder in der Wand der Saugmaske angeordnet.

Bei einer in der WO 2019/008139 A1 gezeigten weiteren Vorrichtung zum Entfernen von Partikeln aus dem Rachen und/oder der sich daran anschließenden Atemwege wird im Anwendungsfall die Beaufschlagung des Maskenteils mit dem Unterdruck eines Unterdruckbereitstellungsteils durch das Ankoppeln des Unterdruckbereitstellungsteils an den Maskenteil ausgelöst.

Bei einer in der WO 2019/239417 A1 vorgeschlagenen weiteren Vorrichtung zum Entfernen eines den Atemweg verschließenden Gegenstands ist eine Vakuumpumpe mit einem federbeaufschlagten Kolben versehen.

In der DE 10 2020 134 455 A1 ist eine Vorrichtung zum Entfernen von Fremdkörpern aus den Atemwegen eines Lebewesens mittels Unterdruck vorgestellt, bei der zwischen einer Behälterwandung und einer Pumpenwandung mit parallelen Wandungsabschnitten ein Druckvolumen zusätzlich zu einem in einer Pumpe gebildeten Pumpenvolumen vorhanden ist. Die Vorrichtung weist mehrere Ventile auf, die in verschiedenen Wandungsabschnitten angeordnet sind.

Der vorliegenden Erfindung liegt die Aufgabe zugrunde, eine Vorrichtung zum Entfernen von den Atemluftweg verschließenden Gegenständen aus dem Rachen, der Luftröhre oder dem anschließendem Atemweg bereitzustellen, die eine gute Handhabbarkeit und Funktionsfähigkeit ergibt.

Diese Aufgabe wird bei einer Vorrichtung mit den Merkmalen des Anspruchs 1 gelöst. Hierbei ist in Verbindung mit den Merkmalen des Oberbegriffs weiterhin vorgesehen, dass die Ablasskanalanordnung einen über den Kolben führenden Ablasskanalabschnitt aufweist, der sich beim Ausziehen des Kolbens zumindest weitgehend schließt und beim Einschieben öffnet.

Mit diesem Aufbau wird beim Einschieben des Kolbens in den vorzugsweise zylindrischen Hohlraum der am Kolben angeordnete Ablasskanalabschnitt geöffnet, sodass die Luft aus dem dem Plenum zugekehrten Hohlraumteil über diesen Ablasskanalabschnitt in die äußere Atmosphäre ungehindert ausströmen kann.

Eine für den Aufbau und die Funktion vorteilhafte Ausgestaltung besteht darin, dass der Kolben einen zentralen Tragkörper aufweist, an dem mittels einer Haltestruktur ein umfangsseitig vollständig umlaufender, den Tragkörper umfangsseitig überragender Kolbenring gehalten ist, der in seinem Außenumfang an den Innenumfang der den Hohlraum umgebenden Wandfläche angepasst ist und beim Verschieben des Kolbens unter Ausübung einer Dichtwirkung entlang der Wandfläche gleitet. Für die so ausgebildete Wirkungsweise des die Strömung in die äußere Atmosphäre zulassenden Einwegventils sind damit keine zusätzlichen Bauelemente erforderlich. Auch wird infolge der dichtenden umfangsseitigen Anlage des Kolbens mit dem Kolbenring an der den Hohlraum umgebenden Wandfläche und der dadurch bewirkten Hemmkraft bzw. durch die Gleitreibungskraft eine sichere Betätigung des Ventils bewirkt, nämlich ein Schließen beim Ausziehen des Kolbens zum Erzeugen des Unterdrucks bzw. Vakuums einerseits und ein Öffnen des Strömungswegs über den Ablasskanalabschnitt beim Einschieben des Kolbens in den Hohlraum andererseits.

Dabei besteht eine für den Aufbau und die Funktion vorteilhafte weitere Ausbildung darin, dass der über den Kolben führende Ablasskanalabschnitt zumindest teilweise zwischen der Außenseite des Tragkörpers und dem dieser benachbarten Flächenbereich des Kolbenrings gebildet ist.

Zu einer vorteilhaften Gestaltung und Funktion der Vorrichtung tragen zudem die Maßnahmen bei, dass die Haltestruktur eine an dem Tragkörper vollständig umlaufende Ringnut aufweist, in der der Kolbenring lose gehalten ist, wobei eine Seitenflanke der Ringnut als vollständig umlaufender, in seiner Querschnittskontur an den Querschnitt eines zugekehrten Abschnitts des Kolbenrings angepasster Dichtsitz ausgebildet ist, in den sich der Kolbenring beim Ausziehen dichtend einschmiegt, um das Vakuum zu bilden, und dass die gegenüberliegende Seitenflanke der Ringnut als weiterer Ringsitz mit mindestens einer Durchströmöffnung versehen ist, durch die der Ablasskanalabschnitt führt, wenn der lose unter Abstand vom Nutgrund in der Ringnut gehaltene Kolbenring beim Einschieben des Kolbens in den weiteren Ringsitz verlagert wird.

Vorteile für den Aufbau und die Bedienung ergeben sich ferner dadurch, dass der Hohlraum in einem Rohrstück gebildet ist und dass das an dem Kolben angebrachte Verbindungsstück eine Kolbenstange ist, die durch einen den Hohlraum auf dessen dem Plenum abgewandter Seite begrenzenden Deckel verschieblich geführt ist.

Eine vorteilhafte Ausgestaltung besteht auch darin, dass die Ablasskanalanordnung über mindestens eine in dem Deckel angeordnete Ausströmöffnung mit der äußeren Atmosphäre in Strömungsverbindung steht.

Unterschiedliche Saugmasken, beispielsweise für Kinder oder Erwachsene, können einfach an der Vakuumpumpe dadurch angebracht werden, dass die Saugmaske z. B. über Dichtmittel abnehmbar mit der Vakuumpumpe verbunden ist.

Bei dem erfindungsgemäßen Aufbau werden eine anwenderfreundliche Ausgestaltung und gute Funktion dadurch erhalten, dass an dem Kolben eine Begrenzungsventileinheit angeordnet ist, die unter Federbelastung einen über den Tragkörper des Kolbens durch mindestens eine Durchlassöffnung führenden Strömungsweg bis zu einem vorgegebenen Grenzdruck geschlossen hält und den Strömungsweg entgegen der Federkraft öffnet, wenn der in dem Plenum erzeugte Unterdruck den Grenzdruck unterschreitet. Auf diese Weise kann unabhängig von der Größe eines Plenums bzw. eines Atemraums die gleiche Vakuumpumpe bzw. Saugmaske bei verschiedenen Personen verwendet werden bzw. die gleiche Vakuumpumpe bei lediglich einer begrenzten Anzahl unterschiedlich großer Saugmasken, da ein zweckmäßig vorgegebener Grenzdruck durch die Wirkung der Begrenzungsventileinheit nicht unterschritten wird. Auch ergibt die Begrenzungsventileinheit eine hohe Benutzerfreundlichkeit bei sicherer Funktion.

Ein vorteilhafter Aufbau der Vorrichtung besteht dabei darin, dass die Begrenzungsventileinheit im Bereich der mindestens einen Durchlassöffnung einen Ventilsitz und einen durch eine Schließfeder bis zum Erreichen des Grenzdrucks in Schließstellung dichtend auf dem Ventilsitz gehaltenen Ventilkegel, insbesondere ausgebildet als Ventilteller, aufweist.

Zu einem vorteilhaften Aufbau und einer guten Funktion trägt ferner bei, dass der Ventilkegel fest mit einem Ventilstößel verbunden ist, der durch eine zentral in dem Tragkörper angeordnete Durchführungsöffnung gegebenenfalls mit einem Führungsfortsatz in eine zugekehrte Sacklochbohrung des als Kolbenstange ausgebildeten Verbindungsstücks eingeführt ist, und dass die Schließfeder als den Ventilstößel umgebende Druckfeder ausgebildet und einerseits auf der von dem Plenum abgekehrten Seite des Tragkörpers gegen diesen und andererseits gegen einen an dem Ventilstößel (am freien Endabschnitt) oder einem mit diesem verbundenen Einsatzstück angeordneten Anschlag abgestützt ist, wobei der Ventilsitz auf der dem Plenum zugekehrten Seite des Tragkörpers angeordnet ist.

Vorteile für den Zusammenbau ergeben sich dabei auch dadurch, dass das Verbindungsstück als separat hergestelltes Teil mit dem Tragkörper axial unverschieblich verbunden ist.

Für den Aufbau und die Montage sind weiterhin die Maßnahmen von Vorteil, dass das Verbindungsstück lösbar mittels einer Halteeinheit mit dem Tragkörper verbunden, insbesondere verrastet oder verschraubt, ist.

Die Erfindung wird nachfolgend anhand von Ausführungsbeispielen unter Bezugnahme auf die Zeichnungen näher erläutert. Es zeigen:
- Fig. 1: eine perspektivische Ansicht einer Vorrichtung zum Entfernen von den Atemluftweg verschließenden Gegenständen mit einer Vakuumpumpe und einer davon abgenommenen Saugmaske in perspektivischer Ansicht,
- Fig. 2A: die Vakuumpumpe nach Fig. 1 in seitlicher Ansicht,
- Fig. 2B: die Vakuumpumpe im Längsschnitt entlang einer Schnittebene A-A nach Fig. 2A,
- Fig. 3: Innenteile der Vakuumpumpe mit Kolbenstange und Kolben aus Tragkörper und Kolbenring in auseinandergenommenem Zustand in perspektivischer Ansicht,
- Fig. 4A: eine weitere Ausführungsform der Vakuumpumpe in seitlicher Ansicht,
- Fig. 4B: die Vakuumpumpe nach Fig. 4A im Längsschnitt entlang der Schnittlinie A-A und
- Fig. 5: Innenteile der Vakuumpumpe in perspektivischer Ansicht bei Ausbildung mit einer Begrenzungsventileinheit.

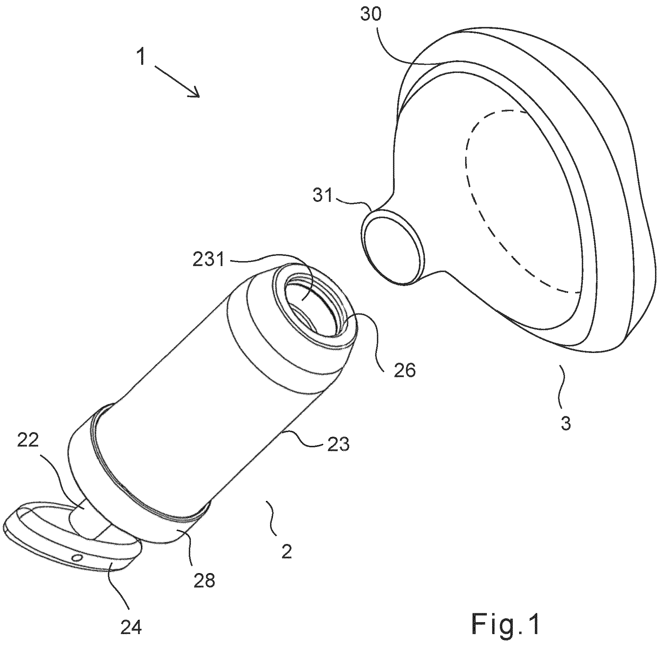

Fig. 1 zeigt eine Vorrichtung zum Entfernen von den Atemluftweg verschließenden Gegenständen mittels Saugwirkung mit einer Saugeinheit 1, die eine Vakuumpumpe 2 und eine daran dicht anschließbare Saugmaske 3 umfasst. Infolge der Austauschbarkeit können Saugmasken unterschiedlicher Art, insbesondere in Form und Größe, an die Vakuumpumpe angeschlossen werden. In alternativer Ausgestaltung kann die Saugmaske auch fest mit der Vakuumpumpe 2 verbunden sein. Sowohl die Vakuumpumpe 2 als auch die Saugmaske 3 bestehen vorteilhaft aus hohe hygienische Anforderungen erfüllendem Kunststoffmaterial.

Die Saugmaske 3 weist ein Abdeckteil 30 mit einem dem Gesicht einer zu behandelnden Person zugekehrten, schmiegsamen Dichtrand auf, mit dem sie über Mund und Nase der Person möglichst gut dichtend aufgesetzt werden kann, und ist auf der von dem Dichtrand abgekehrten Seite des Abdeckteils 30 mit einem daran angeformten, relativ steifen Anschlussstück 31 versehen, mit dem sie in oder über eine Öffnung in einem Saugmund 231 der Vakuumpumpe 2 unter Abdichtung einsteckbar bzw. aufsteckbar ist. In dem Saugmund 231 ist bei dem gezeigten Ausführungsbeispiel ein Dichtring 26, beispielsweise O-Ring, angeordnet.

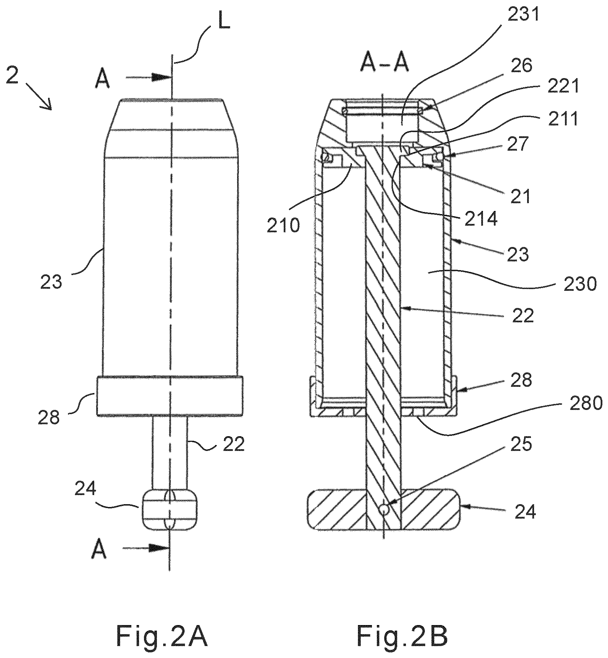

Die Vakuumpumpe 2 weist bei dem gezeigten Ausführungsbeispiel, wie auch aus den Fig. 2A und 2B ersichtlich, einen längserstreckten, zylinderförmigen, insbesondere im Querschnitt kreisförmigen, Tubus auf, der einen Hohlraum 230 umfangsseitig umschließt und z. B. als Rohrstück 23 ausgeführt ist, das sich mit seiner Umfangswandung konzentrisch um eine Mittellängsachse L erstreckt. Der Tubus bzw. das Rohrstück 23 ist an seinem der Saugmaske 3 zugekehrten einen, dem vorderen, Ende mit einem den Saugmund 231 umfassenden Endstück 232 versehen und an seinem anderen, dem hinteren, Endbereich, mit einem fest oder abnehmbar angebrachten Deckel 28 abgedeckt.

In dem Hohlraum 230 ist ein Kolben 21 in Richtung der Längsachse L verschieblich aufgenommen, der entlang der den Hohlraum umgebenden Wandfläche, vorliegend also der inneren Wandfläche des Tubus bzw. Rohrstücks 23, entlang gleitend, (relativ) luftdicht geführt ist, um in einem Anwendungsfall in dem der Saugmaske 3 zugekehrten Hohlraumteil und dem strömungstechnisch anschließenden Innenraum der Saugmaske 3 und dem Atemwegraum bzw. dem dadurch gebildeten Plenum eine genügend hohe Saugwirkung zu erzielen, sodass ein in den Atemluftweg gelangter Gegenstand durch die Saugwirkung möglichst gut entfernt werden kann.

Wie insbesondere Fig. 2B zeigt, ist der Kolben 21 mit einem konzentrisch zur Mittellängsachse L in dem Hohlraum 230 verlaufenden Kolbenstange 22 verbunden, die sich auf der von dem Saugmund 231 abgekehrten Seite des Kolbens 21 erstreckt und durch eine zentrale Öffnung des Deckels 28 verschieblich geführt ist und an ihrem außerhalb des Deckels 28 liegenden Ende mit einem Griff 24 versehen ist, der z. B. mittels eines Haltestifts 25 fixiert ist und als Handhabe für die manuelle Verschiebung des Kolbens 21 dient. Kolbenseitig ist die Kolbenstange 22 durch eine zentrale Bohrung bzw. Durchführung 214 in einem Tragekörper 210 des Kolbens 21 geführt und in einer verbreiterten Ausnehmung 211 auf der Saugmundseite des Kolbens 231 mittels eines Halteflanschs 221 festgelegt, sodass sich der Kolben zum Herausziehen und Ausüben der Saugwirkung stabil und gut geführt verschieben lässt.

Damit beim Einschieben des Kolbens in Richtung auf die Saugmaske 3 im Anwendungsfall kein erhöhter Luftdruck in dem Plenum und damit dem Atemluftweg erzeugt wird, der den im Atemluftweg befindlichen Gegenstand noch weiter hineinbewegen könnte, ist in der Saugeinheit 1 und zwar vorliegend in der Vakuumpumpe 2, eine Ablasskanalanordnung ausgebildet, durch die die beim Einschieben des Kolbens 21 in dem Plenum verdrängte Luft in die äußere Atmosphäre möglichst ungehindert abströmen kann. Hierzu ist die Ablasskanalanordnung mit einem über den Kolben 21 führenden Ablasskanalabschnitt ausgebildet, der sich beim Ausziehen des Kolbens 21 zum Erzeugen eines möglichst gut wirksamen Vakuums schließt und beim Einschieben zum Durchlassen der im Plenum verdrängten Luft öffnet. Insbesondere ist der Ablasskanalabschnitt zwischen der Umfangsseite des Tragkörpers 210 des Kolbens 21 und einem auf diesem gehaltenen, ihn vollständig umgebenden Kolbenring 27 gebildet, der mit seinem äußeren Umfangsflächenabschnitt (relativ) gut dichtend an der den Hohlraum umgebenden Wandfläche entlang gleitet. Der Kolbenring 27 steht mit seinem radial äußeren umlaufenden Bereich über den radial äußeren umlaufenden Randbereich des Tragkörpers 210 radial vor. Der Kolbenring 27 ist z. B. als O-Ring mit (in radialer Richtung, senkrecht zur Ringebene verlaufendem) rundem, insbesondere kreisförmigem Querschnitt ausgeführt; jedoch kommen auch andere Querschnittsformen in Betracht.

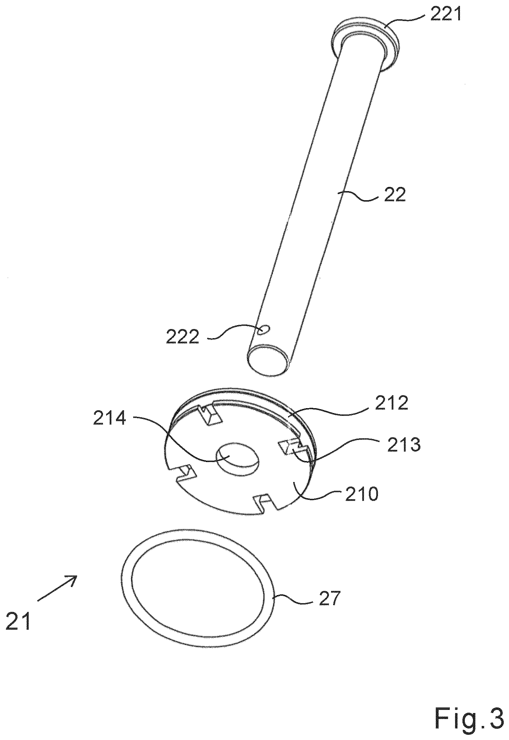

Wie Fig. 3 genauer zeigt, ist der z. B. scheibenförmig ausgebildete Tragkörper 210 zentral mit der Durchführung 214 für die Kolbenstange 22 versehen und weist als Haltestruktur für den Kolbenring 27 eine vollständig umlaufende Ringnut 212 auf, die auf ihrer dem Saugmund 231 bzw. dem zu bildenden Plenum zugewandten Seite mit einer vollständig umlaufenden Seitenflanke ausgebildet ist und auf ihrer dem Deckel 28 zugewandten anderen Seite mit einer mehrere Durchströmöffnungen 213 aufweisenden Seitenflanke versehen ist. Der Kolbenring 27 ist in der Ringnut 212 lose gehalten, wobei der Innenumfang des Kolbenrings 27 etwas größer ist als der Außenumfang des Tragkörpers 210 im Nutgrund der Ringnut 212, aber kleiner als der radial äußere Umfangsrand der beiden Seitenflanken der Ringnut 212. Zudem ist der Durchmesser des Kolbenrings 27 in axialer Richtung (in Richtung der Mittellängsachse L) kleiner als die axiale Breite der Ringnut 212 im Bereich der Seitenflanken, wodurch der Kolbenring 27 lose in der Ringnut 212 gehalten ist. Vorteilhaft ist die eine, dem Saugmund 231 zugekehrten Seitenflanke der Ringnut 212 in ihrer Querschnittskontur an die zugekehrte Teilquerschnittskontur des Kolbenrings 27 so angepasst, dass sich der Kolbenring 27 beim Ausziehen des Kolbens 21 in diese Seitenflanke gut dichtend einschmiegen kann.

Während sich der Kolbenring 27 beim Ausziehen des Kolbens 21 zum Bilden eines möglichst guten Vakuums im Plenum im Anwendungsfalle in der Ringnut 212 dichtend anschmiegt und dichtend an der umgebenden Wandfläche des Hohlraums entlang gleitet, bewegt sich der Kolbenring 27 infolge seines losen Sitzes und der Hemmkraft an der den Hohlraum umgebenden Wandfläche bzw. infolge der dadurch bewirkten Gleitreibung zur anderen Seitenflanke der Ringnut 212 hin, wodurch der so gebildete Ablasskanalabschnitt beim Einschieben des Kolbens 21 öffnet, sodass im Plenum verdrängte Luft zwischen der saugmundseitigen Seitenflanke und dem Nutgrund der Ringnut sowie durch die Durchströmöffnungen 213 ausströmen und über weitere Ausströmöffnungen 280 auf der von dem Plenum abgewandten Seite des Kolbens 21 in die äußere Atmosphäre ausströmen kann, sodass in dem Plenum beim Einschieben des Kolbens 21 praktisch kein Staudruck auftritt.

Die beschriebene Ausgestaltung der Ablasskanalanordnung mit dem Ablasskanalabschnitt besitzt bei guter Funktionsfähigkeit auch den Vorteil, dass zum Erzeugen der angegebenen Einwegventilwirkung keine zusätzlichen Bauteile für den Aufbau der Vakuumpumpe erforderlich sind. Denkbar wäre in alternativer Ausgestaltung auch ein Einbau eines zusätzlichen Einwegventils in dem Kolben 21, insbesondere dem Tragkörper 210, wie z. B. in Gestalt eines oder mehrerer Entenschnabelventile oder anderer Betätigungselemente, die aber erst bei sich aufbauendem Druck im Plenum öffnen und daher unter Umständen, z. B. bei Verklebung, weniger gut öffnen bzw. wirksam sind.

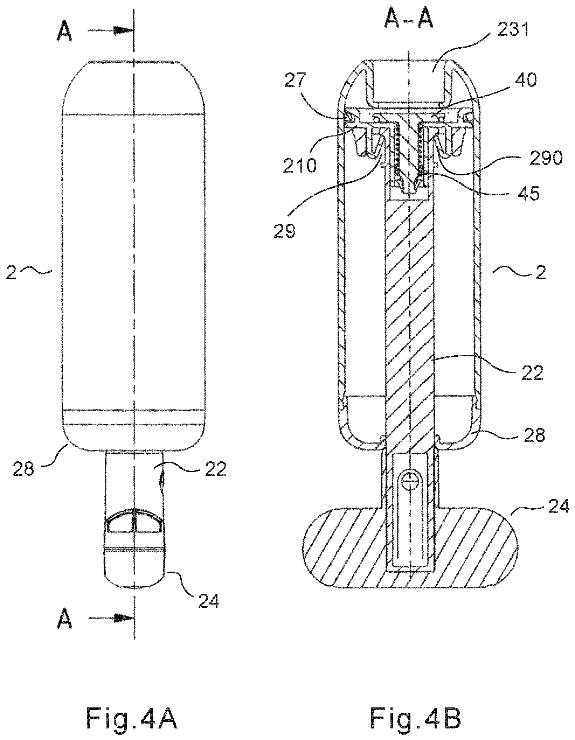

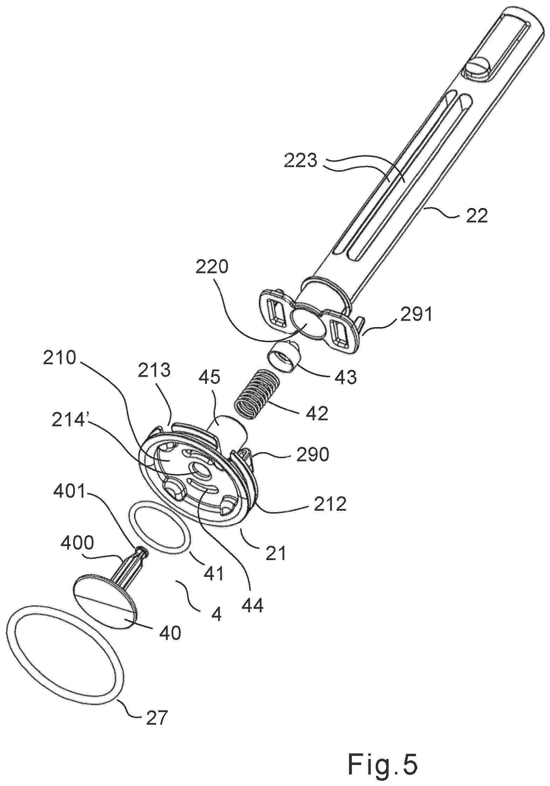

In weiterer Ausgestaltung ist die Saugeinheit 1 der Vorrichtung, insbesondere die Vakuumpumpe 2 mit einer Begrenzungsventileinheit 4 versehen, wie in den Fig. 4A, 4B und 5 gezeigt.

Fig. 4A zeigt eine abgewandelte Form der Vakuumpumpe 2 gegenüber dem Ausführungsbeispiel nach Fig. 2A, wobei der den Tubus bzw. das Rohrstück 23 gegenüber der Saugmaskenseite abdeckende Deckel 28 zur Oberseite gerundet ausgeführt ist. Fig. 4B zeigt einen Längsschnitt der Ausführung nach Fig. 4A entlang einer Schnittebene A-A. Der wesentliche Unterschied gegenüber der vorhergehend beschriebenen Ausführungsform ist hierbei, dass in den Kolben 21, und zwar in den Tragkörper 210 und den daran angeschlossenen Abschnitt des Verbindungsstücks 22 in Form der Kolbenstange eine Begrenzungsventileinheit 4 eingebaut ist. Die über den Kolben 21 führende Ablasskanalanordnung mit der umlaufenden Ringnut 212 und dem darin lose gehaltenen Kolbenring 27 mit der Ausbildung der Seitenflanken zum Bilden der Dichtsitze beim Einschieben und Ausziehen des Kolbens 21 mit Ausbildung der mindestens einen Durchströmöffnung 213 ist vorzugsweise entsprechend der vorher beschriebenen Ausführungsform gemäß den Fig. 1 bis 3 ausgebildet. Jedoch kann die Begrenzungsventileinheit 4 an sich auch in einem Kolben 21 und Verbindungsstück 22 im Zusammenhang mit einer anderen Ausbildung des Ablasskanalabschnitts bzw. der Ablasskanalanordnung verwendet werden, da sie davon unabhängig ausgebildet ist.

Wie aus den Fig. 4B und 5 ersichtlich, weist die Begrenzungsventileinheit 4 auf der dem Plenum bzw. dem zu erzeugenden Vakuumraum zugewandten Seite des Tragkörpers 210 einen, insbesondere kreisrunden, Ventilteller 40 auf, der mit einem daran zentral angebrachten, insbesondere angeformten, Ventilstößel 400 auf der von dem Plenum abgewandten Seite des Ventiltellers 40 versehen ist. Der Ventilstößel 400 durchragt eine zentral in dem Tragkörper 210 eingebrachte Durchführungsöffnung 214'. Auf der von dem Plenum abgekehrten Rückseite des Tragkörpers 210 ist um die zentrale Durchführungsöffnung 214' ein hülsenförmiger Ansatz bzw. Fortsatz 45 angeformt, mit dem der Tragkörper 210 in eine zugewandte Sacklochbohrung 220 in dem zugekehrten Abschnitt des Verbindungsstücks 22 in Form der Kolbenstange passgenau einsetzbar ist, wobei also die Querschnittskontur der Sacklochbohrung 220 mit ihrer umgebenden Wandfläche an die Außenkontur des hülsenartigen Fortsatzes 45 des Ventiltellers 40 angepasst ist. In die Sacklochbohrung 220 bzw. durch den hülsenförmigen Fortsatz 45 ist der Ventilstößel 400 koaxial eingeführt und mittels eines von dem Ventilteller 40 abgekehrten endseitigen Halteabschnitts 401 an einem in die Sacklochbohrung 220 eingesetzten, axial verschieblichen Einsatzstück 43 (gegenüber diesem axial unverschieblich) gehalten, z. B. verrastet, eingeklipst oder eingeschraubt. Dabei ist der Ventilstößel 401 von einer Schließfeder 42 in Form einer Druckfeder umgeben, die sich einerseits innerhalb des Fortsatzes 45 an einem in dem Hohlraum vorhandenen bzw. um die Durchführungsöffnung 214' verlaufenden Stützrand des Tragkörpers 210 und andererseits gegen einen Anschlag des Ventilstößels 400 im Bereich des Halteabschnitts 401 bzw. innerhalb des Einsatzstücks 43 abstützt. Der Tragkörper 210 bzw. der Kolben 21 mit dem Tragkörper 210 ist mittels einer Halteeinheit 29, welche einerseits mindestens ein an der von dem Plenum abgewandten Seite des Tragkörpers 210 angebrachtes, insbesondere angeformtes, Halteelement und andererseits ein damit zusammenwirkendes, an dem zugewandten Abschnitt des Verbindungsstücks 22 angebrachtes, insbesondere angeformtes, Haltegegenelement 291 aufweist, stabil an dem Verbindungsstück 22 festgehalten. Das Halteelement 290 kann z. B. als Schnapphaken und das Haltegegenelement 291 als Haltenase bzw. Haltevorsprung ausgebildet sein oder umgekehrt. Somit kann die Begrenzungsventileinheit 4 mit dem Ventilteller 40 und Ventilstößel 400 sowie der Schließfeder und dem Einsatzstück 43 an dem Verbindungsstück 22 bei einfacher Montage eingesetzt und mittels der Halteeinheit 29 an dem Verbindungsstück 22 stabil und gegebenenfalls abnehmbar gehalten werden.

Der Ventilteller 40 bildet eine Art Ventilkegel, der mit einem auf der dem Plenum zugewandten Seite des Tragkörpers 210 angeordneten Ventilsitz dichtend zusammenwirkt. Dabei ist in dem Tragkörper 210 mindestens eine Durchlassöffnung 44 ausgebildet, durch die bei von dem Ventilsitz abgehobenem Ventilteller 40 ein Strömungsweg in den Tubus bzw. das Rohrstück der Saugpumpe gebildet wird und somit ein Strömungsweg zum Begrenzen des in dem Plenum erzeugten Unterdrucks gebildet ist. Eine Verbindung zur äußeren Atmosphäre wird bei dieser Ausführung über Längsöffnungen 223 in dem Verbindungsstück 22 bzw. der Kolbenstange hergestellt. Alternativ kann auch hierbei am Tubus der Vakuumpumpe z. B. über den Deckel 28 und eine Ausströmöffnung 230 eine Verbindung zur äußeren Atmosphäre hergestellt werden. Zum guten Abdichten zwischen Ventilteller 40 und Ventilsitz an dem Tragkörper 210 ist um die mindestens eine Durchlassöffnung 44 ein Dichtungselement 41 vorhanden, gegen die die von dem Plenum abgewandte Innenseite des Ventiltellers 40 mittels der Schließfeder 42 mit einer vorgegebenen Federkraft bzw. Schließkraft angedrückt wird.

Wird in dem Plenum ein Unterdruck erzeugt, der einen durch die Federkraft bewirkten, vorgegebenen Grenzdruck unterschreitet, öffnet die Begrenzungsventileinheit 4, indem der Ventilteller 40 vom Ventilsitz abgehoben wird und ein Strömungsweg über die mindestens eine Durchlassöffnung 44 geöffnet wird. Damit wird ein vorgebbarer Unterdruck nicht unterschritten und die Vakuumpumpe kann bei unterschiedlichen Größen des Plenums bzw. unterschiedlichen Atemräumen und verwendeten Saugmasken 3 in gleicher Funktionsweise verwendet werden, ohne dass ein zu hoher Unterdruck beim Herausziehen der Kolbenstange 22 zum Entfernen eines Fremdkörpers aus dem Atemweg erzeugt wird. Ein üblicher Saugdruck liegt z. B. zwischen 0,3 bar und 0,6 bar.

Die erfindungsgemäßen Maßnahmen ergeben insbesondere auch in Verbindung mit der Begrenzungsventileinheit 4 einen benutzerfreundlichen Aufbau mit zuverlässiger Funktionsweise.

## Patentansprüche

1. Vorrichtung zum Entfernen von den Atemluftweg verschließenden Gegenständen mittels Saugwirkung, mit einer auf das Gesicht aufsetzbaren, den Mund und/oder die Nase zumindest weitgehend dicht umgebenden Saugmaske (3) und einer an diese zum Bilden einer Saugeinheit (1) dicht angeschlossenen oder anschließbaren, manuell betätigbaren Vakuumpumpe (2), die einen in einem Hohlraum (230) entlang einer diesen umgebenden Wandfläche geführten, von Hand mittels eines Verbindungsstücks (22) verschiebbaren Kolben (21) aufweist, der beim Einschieben in den Hohlraum (230) Luft aus dem Hohlraum (230) über eine in die äußere Atmosphäre führende Ablasskanalanordnung verdrängt und beim Ausziehen im Hohlraum (230) bei angelegter Saugmaske (3) Unterdruck im Plenum aus einem dieser zugekehrten Hohlraumteil, Saugmaskeninnenraum und anschließendem Atemluftweg erzeugt,
**dadurch gekennzeichnet,**
**dass** die Ablasskanalanordnung einen über den Kolben (21) führenden Ablasskanalabschnitt aufweist, der sich beim Ausziehen des Kolbens (21) zumindest weitgehend schließt und beim Einschieben öffnet, und
**dass** an dem Kolben (21) eine Begrenzungsventileinheit (4) angeordnet ist, die unter Federbelastung einen über den Tragkörper (210) des Kolbens (21) durch mindestens eine Durchlassöffnung (44) führenden Strömungsweg bis zu einem vorgegebenen Grenzdruck geschlossen hält und den Strömungsweg entgegen der Federkraft öffnet, wenn der in dem Plenum erzeugte Unterdruck den Grenzdruck unterschreitet.

2. Vorrichtung nach Anspruch 1,
**dadurch gekennzeichnet,**
**dass** der Kolben (21) einen zentralen Tragkörper (210) aufweist, an dem mittels einer Haltestruktur ein umfangsseitig vollständig umlaufender, den Tragkörper (210) umfangsseitig überragender Kolbenring (27) gehalten ist, der in seinem Außenumfang an den Innenumfang der den Hohlraum (230) umgebenden Wandfläche angepasst ist und beim Verschieben des Kolbens (21) unter Ausübung einer Dichtwirkung entlang der Wandfläche gleitet.

3. Vorrichtung nach Anspruch 2,
**dadurch gekennzeichnet,**
**dass** der über den Kolben (21) führende Ablasskanalabschnitt zumindest teilweise zwischen der Außenseite des Tragkörpers (210) und dem dieser benachbarten Flächenbereich des Kolbenrings (27) gebildet ist.

4. Vorrichtung nach Anspruch 2 oder 3,
**dadurch gekennzeichnet,**
**dass** die Haltestruktur eine an dem Tragkörper (210) vollständig umlaufende Ringnut (212) aufweist, in der der Kolbenring (27) lose gehalten ist, wobei eine Seitenflanke der Ringnut (212) als vollständig umlaufender, in seiner Querschnittskontur an den Querschnitt eines zugekehrten Abschnitts des Kolbenrings (27) angepasster Dichtsitz ausgebildet ist, in den sich der Kolbenring (27) beim Ausziehen dichtend einschmiegt, um das Vakuum zu bilden, und
**dass** die gegenüberliegende Seitenflanke der Ringnut (212) als weiterer Ringsitz mit mindestens einer Durchströmöffnung (213) versehen ist, durch die der Ablasskanalabschnitt führt, wenn der lose unter Abstand vom Nutgrund in der Ringnut (212) gehaltene Kolbenring (27) beim Einschieben des Kolbens (21) in den weiteren Ringsitz verlagert wird.

5. Vorrichtung nach einem der vorhergehenden Ansprüche,
**dadurch gekennzeichnet,**
**dass** der Hohlraum (230) in einem Rohrstück (23) gebildet ist und
**dass** das an dem Kolben (21) angebrachte Verbindungsstück (22) eine Kolbenstange ist, die durch einen den Hohlraum (230) auf dessen dem Plenum abgewandter Seite begrenzenden Deckel (28) verschieblich geführt ist.

6. Vorrichtung nach Anspruch 5,
**dadurch gekennzeichnet,**
**dass** die Ablasskanalanordnung über mindestens eine in dem Deckel (28) angeordnete Ausströmöffnung (280) mit der äußeren Atmosphäre in Strömungsverbindung steht.

7. Vorrichtung nach einem der vorhergehenden Ansprüche,
**dadurch gekennzeichnet,**
**dass** die Saugmaske (3) über Dichtmittel abnehmbar mit der Vakuumpumpe (2) verbunden ist.

8. Vorrichtung nach einem der vorhergehenden Ansprüche,
**dadurch gekennzeichnet,**
**dass** die Begrenzungsventileinheit (4) im Bereich der mindestens einen Durchlassöffnung (44) einen Ventilsitz und einen durch eine Schließfeder (42) bis zum Erreichen des Grenzdrucks in Schließstellung dichtend auf dem Ventilsitz gehaltenen Ventilkegel, insbesondere ausgebildet als Ventilteller (40), aufweist.

9. Vorrichtung nach Anspruch 8,
**dadurch gekennzeichnet,**
**dass** der Ventilkegel fest mit einem Ventilstößel (400) verbunden ist, der durch eine zentral in dem Tragkörper (210) angeordnete Durchführungsöffnung (214') in eine zugekehrte Sacklochbohrung (220) des als Kolbenstange ausgebildeten Verbindungsstücks (22) eingeführt ist, und
**dass** die Schließfeder (42) als den Ventilstößel (400) umgebende Druckfeder ausgebildet und einerseits auf der von dem Plenum abgekehrten Seite des Tragkörpers (210) gegen diesen und andererseits gegen einen an dem Ventilstößel (400) oder an einem mit diesem axial unverschieblich verbundenen Einsatzstück (43) angeordneten Anschlag abgestützt ist, wobei der Ventilsitz auf der dem Plenum zugekehrten Seite des Tragkörpers (210) angeordnet ist.

10. Vorrichtung nach einem der vorhergehenden Ansprüche,
**dadurch gekennzeichnet,**
**dass** das Verbindungsstück (22) als separat hergestelltes Teil mit dem Tragkörper (210) verbunden ist.

11. Vorrichtung nach Anspruch 10,
**dadurch gekennzeichnet,**
**dass** das Verbindungsstück (22) lösbar mittels einer Halteeinheit (29) mit dem Tragkörper (210) verbunden, insbesondere verrastet oder verschraubt, ist.

## Claims

1. Device for removing objects obstructing the airway by means of suction, the device comprising a suction mask (3) which is placeable on the face and surrounds the mouth and/or the nose in an at least largely sealed manner and a manually actuatable vacuum pump (2) which is attached or attachable to said mask in a sealed manner in order to form a suction unit (1) and has a piston (21) guided in a cavity (230) along a wall surface which surrounds said cavity and movable by hand by means of a connecting piece (22), which piston, when pushed into the cavity (230), displaces air from the cavity (230) via an outlet channel arrangement leading to the outside atmosphere and, when pulled out of the cavity (230) with the suction mask (3) in place, generates negative pressure in the plenum from a cavity part near said mask, from the suction mask interior, and from the adjoining airway,
**characterized**
**in that** the outlet channel arrangement has an outlet channel portion leading over the piston (21), which portion at least largely closes when the piston (21) is pulled out and opens when it is pushed in, and
**in that** on the piston (21) is arranged a limiting valve unit (4) which, under spring load, keeps a flow path, leading over the support body (210) of the piston (21) through at least one passage opening (44), closed up to a predetermined limit pressure and opens the flow path against the spring force when the negative pressure generated in the plenum is below the limit pressure.

2. Device according to claim 1,
**characterized**
**in that** the piston (21) has a central support body (210) on which a circumferentially completely encircling piston ring (27) circumferentially extending beyond the support body (210) is held by means of a holding structure, which ring is adapted in its outer circumference to the inner circumference of the wall surface surrounding the cavity (230) and, when the piston (21) is moved, slides along the wall surface to exert a sealing effect.

3. Device according to claim 2,
**characterized**
**in that** the outlet channel portion leading over the piston (21) is formed at least partially between the outside of the support body (210) and the adjacent surface region of the piston ring (27).

4. Device according to claim 2 or 3,
**characterized**
**in that** the holding structure has on the support body (210) a completely encircling annular groove (212) in which the piston ring (27) is loosely held, wherein a side flank of the annular groove (212) is in the form of a completely encircling sealing seat which is adapted in its contour to the cross section of a near portion of the piston ring (27) and into which, when the piston is pulled out, the piston ring (27) sealingly fits in order to form the vacuum, and
**in that** the opposite side flank of the annular groove (212) is provided as a further ring seat comprising at least one through-flow opening (213) through which the outlet channel portion leads when the piston ring (27) held loosely in the ring groove (212) at a distance from the groove base is displaced into the further ring seat as the piston (21) is pushed in.

5. Device according to any of the preceding claims,
**characterized**
**in that** the cavity (230) is formed in a pipe piece (23) and in that the connecting piece (22) attached to the piston (21) is a piston rod which is movably guided by a lid (28) limiting the cavity (230) on its side remote from the plenum.

6. Device according to claim 5,
**characterized**
**in that** the outlet channel arrangement is fluidically connected to the outside atmosphere via at least one outflow opening (280) arranged in the lid (28).

7. Device according to any of the preceding claims,
**characterized**
**in that** the suction mask (3) is detachably connected to the vacuum pump (2) via sealing means.

8. Device according to any of the preceding claims,
**characterized**
**in that** the limiting valve unit (4) has in the region of the at least one passage opening (44) a valve seat and a valve cone, in particular in the form of a valve disk (40), which in a closed position is held sealingly on the valve seat by a closing spring (42) until the limit pressure is reached.

9. Device according to claim 8,
**characterized**
**in that** the valve cone is fixedly connected to a valve tappet (400) which is inserted through a passage opening (214') arranged centrally in the support body (210), into a near blind bore (220) of the connecting piece (22) in the form of a piston rod, and
**in that** the closing spring (42) is in the form of a compression spring surrounding the valve tappet (400) and is supported on the side of the support body (210) facing away from the plenum against said support body and against a stop arranged on the valve tappet (400) or on an insert piece (43) axially immovably connected thereto, wherein the valve seat is arranged on the side of the support body (210) facing the plenum.

10. Device according to any of the preceding claims,
**characterized**
**in that** the connecting piece (22) is connected to the support body (210) as a separately manufactured part.

11. Device according to claim 10,
**characterized**
**in that** the connecting piece (22) is releasably connected, in particular latched or screwed, to the support body (210) by means of a holding unit (29).

## Revendications

1. Dispositif permettant d'éliminer par aspiration des objets obstruant le trajet d'air respirable, comportant un masque d'aspiration (3) pouvant être posé sur le visage et entourant la bouche et/ou le nez de manière au moins largement étanche, et une pompe à vide (2) actionnable manuellement, raccordée ou pouvant être raccordée de manière étanche audit masque pour former une unité d'aspiration (1), laquelle pompe à vide présente un piston (21) guidé dans une cavité (230) le long d'une surface de paroi entourant ladite cavité et pouvant être déplacé manuellement au moyen d'une pièce de liaison (22), lequel piston, lorsqu'il est introduit dans la cavité (230), refoule l'air hors de la cavité (230) par l'intermédiaire d'un agencement formant canal d'évacuation menant à l'atmosphère extérieure et, lorsqu'il est retiré de la cavité (230) et que le masque d'aspiration (3) est mis en place, produit une dépression dans le plénum formé à partir d'une partie de cavité tournée vers celui-ci, de l'espace intérieur de masque d'aspiration et du trajet d'air respirable qui s'y raccorde,
**caractérisé en ce**
**que** l'agencement formant canal d'évacuation présente une section de canal d'évacuation passant par le piston (21), laquelle section se ferme au moins en grande partie lors du retrait du piston (21) et s'ouvre lors de l'insertion, et
**qu'**est disposée sur le piston (21) une unité formant soupape de limitation (4) qui, sous la charge d'un ressort, maintient fermée un trajet d'écoulement passant par le corps de support (210) du piston (21) à travers au moins une ouverture de passage (44) jusqu'à une pression limite prédéfinie et ouvre le trajet d'écoulement à l'encontre de la force du ressort lorsque la dépression générée dans le plénum n'atteint pas la pression limite.

2. Dispositif selon la revendication 1,
**caractérisé en ce**
**que** le piston (21) présente un corps de support (210) central sur lequel est maintenu, au moyen d'une structure de maintien, un segment de piston (27) s'étendant entièrement sur la périphérie, faisant saillie au-delà du corps de support (210) sur la périphérie et dont la périphérie extérieure est adaptée à la périphérie intérieure de la surface de paroi entourant la cavité (230) et lequel glisse le long de la surface de paroi lors du déplacement du piston (21) en exerçant un effet d'étanchéité.

3. Dispositif selon la revendication 2,
**caractérisé en ce**
**que** la section de canal d'évacuation passant par le piston (21) est formée au moins partiellement entre le côté extérieur du corps de support (210) et la zone de surface du segment de piston (27) voisine de celui-ci.

4. Dispositif selon la revendication 2 ou 3,
**caractérisé en ce**
**que** la structure de maintien présente une rainure annulaire (212) s'étendant entièrement sur le corps de support (210), dans laquelle le segment de piston (27) est maintenu de manière lâche, dans lequel un flanc latéral de la rainure annulaire (212) est réalisé sous la forme d'un siège d'étanchéité s'étendant entièrement sur la périphérie et dont le contour de section transversale est adapté à la section transversale d'une section faisant face du segment de piston (27), dans lequel siège le segment de piston (27) s'introduit de manière étanche lors du retrait afin de former le vide, et
**que** le flanc latéral opposé de la rainure annulaire (212) est pourvu, en tant que siège annulaire supplémentaire, d'au moins une ouverture de passage (213) à travers laquelle passe la section de canal d'évacuation lorsque le segment de piston (27) maintenu de manière lâche dans la rainure annulaire (212) à distance du fond de rainure est déplacé lors de l'insertion du piston (21) dans le siège annulaire supplémentaire.

5. Dispositif selon l'une des revendications précédentes,
**caractérisé en ce**
**que** la cavité (230) est formée dans une pièce tubulaire (23) et que la pièce de liaison (22) montée sur le piston (21) est une tige de piston qui est guidée de manière à pouvoir coulisser à travers un moyen de recouvrement (28) délimitant la cavité (230) sur son côté opposé au plénum.

6. Dispositif selon la revendication 5,
**caractérisé en ce**
**que** l'agencement formant canal d'évacuation est en liaison par écoulement avec l'atmosphère extérieure par l'intermédiaire d'au moins une ouverture d'échappement (280) disposée dans le moyen de recouvrement (28).

7. Dispositif selon l'une des revendications précédentes,
**caractérisé en ce**
**que** le masque d'aspiration (3) est relié de manière amovible à la pompe à vide (2) par l'intermédiaire de moyens d'étanchéité.

8. Dispositif selon l'une des revendications précédentes,
**caractérisé en ce**
**que** l'unité formant soupape de limitation (4) présente, dans la zone de l'au moins une ouverture de passage (44), un siège de soupape et un cône de soupape, en particulier réalisé sous forme de tête de soupape (40), maintenu de manière étanche sur le siège de soupape par un ressort de fermeture (42) jusqu'à l'atteinte de la pression limite dans la position de fermeture.

9. Dispositif selon la revendication 8,
**caractérisé en ce**
**que** le cône de soupape est relié fixement à un poussoir de soupape (400) qui est introduit par une ouverture de passage (214') disposée au centre dans le corps de support (210) dans un alésage borgne (220) faisant face de la pièce de liaison (22) réalisée sous forme de tige de piston, et
**que** le ressort de fermeture (42) est réalisé sous forme de ressort de pression entourant le poussoir de soupape (400) et s'appuie d'une part, sur le côté du corps de support (210) opposé au plénum, contre celui-ci et d'autre part contre une butée disposée sur le poussoir de soupape (400) ou sur une pièce formant insert (43) reliée à celui-ci de manière à ne pas pouvoir coulisser axialement, dans lequel le siège de soupape est disposé sur le côté du corps de support (210) faisant face au plénum.

10. Dispositif selon l'une des revendications précédentes,
**caractérisé en ce**
**que** la pièce de liaison (22) est reliée au corps de support (210) en tant que pièce fabriquée séparément.

11. Dispositif selon la revendication 10,
**caractérisé en ce**
**que** la pièce de liaison (22) est reliée, en particulier encliquetée ou vissée, de manière amovible au corps de support (210) au moyen d'une unité de maintien (29).
